# FASCICULE DE BREVET EUROPEEN

(11) **EP 2 198 036 B1**
(45) Date de publication et mention de la délivrance du brevet: **21.12.2011**
(21) Numéro de dépôt: 08836942.6
(22) Date de dépôt: 17.09.2008
(51) Int. Cl.: C12P 7/40, C12P 7/62, C07C 51/353, C07C 57/03, C07C 67/333, C07C 69/533

(54) **PROCEDE DE SYNTHESE D'ACIDES GRAS OMEGA-INSATURES**
VERFAHREN ZUR SYNTHESE UNGESÄTTIGTER OMEGA-FETTSÄUREN
METHOD FOR THE SYNTHESIS OF OMEGA-UNSATURATED FATTY ACIDS

(30) Priorité: 20.09.2007 FR 0757694
(43) Date de publication de la demande: 23.06.2010
(73) Titulaire: ARKEMA FRANCE, 92700 Colombes (FR)
(72) Inventeur: DUBOIS, Jean-Luc, F-69390 Millery (FR)
(74) Mandataire: Sauvageot, Olivier Roger
(86) Numéro de dépôt international: PCT/FR2008/051665
(87) Numéro de publication internationale: WO 2009/047445

(56) Documents cités:
- EP-A- 0 444 264
- US-A- 4 474 882
- WARWEL S ET AL: "Polymers and surfactants on the basis of renewable resources" CHEMOSPHERE, PERGAMON PRESS, OXFORD, GB, vol. 43, 1 janvier 2001 (2001-01-01), pages 39-48, XP002262684 ISSN: 0045-6535
- J.C. MOL: "Catalytic metathesis of unsaturated fatty acid esters and oils" TOPICS IN CATALYSIS, vol. 27, no. 1-4, 2004, pages 97-104, XP002481667 cité dans la demande
- C.-X. BAI ET AL.: "Lewis-acid assisted cross metathesis of acrylonitrile with functionalized olefins catalyzed by phosphine-free ruthenium carbonen complex" ORG. BIOMOL. CHEM., vol. 3, 2005, pages 4139-4142, XP002481668 cité dans la demande

## Description

L'invention vise un procédé de synthèse par métathèse d'acides ou d'esters gras ω-insaturés à partir d'un acide gras ou d'un ester gras mono-insaturé naturel.

Les acides ou d'esters gras ω-insaturés sont des molécules utiles dans toute une série de réactions telles que la carbonylation, l'hydroformylation et l'époxydation notamment. Ces acides ne sont généralement pas préparés selon les procédés de synthèse chimique habituels des acides insaturés qui conduisent généralement à des acides α ou β insaturés et non à des acides gras ω-insaturés. Donc en pratique ce type d'acides provient de la transformation d'acides insaturés plus complexes et notamment des acides gras d'origine naturelle, qui sont soumis à des traitements plus ou moins complexes.

C'est ainsi qu'est connu un procédé industriel de fabrication de l'acide amino-11-undécanoique utilisant comme matière première l'acide ricinoléique extrait de l'huile de ricin, qui est à la base de la synthèse du Rilsan^{®}. Dans ce procédé l'acide ω-undécylénique, sous sa forme ester, undécylénate, est synthétisé à titre de produit intermédiaire par pyrolyse de la charge qui s'accompagne de la formation comme co-produit de l'heptanaldéhyde. Ce procédé est décrit dans l'ouvrage « Les Procédés de Pétrochimie » de A. Chauvel et al. paru aux Editions TECHNIP (1986).

Une autre méthode de synthèse des acides ou d'esters gras ω-insaturés à « chaîne courte » à partir d'acides ou d'esters gras à « longue chaîne » consiste à effectuer une métathèse croisée de ces acides à longue chaîne avec l'éthylène, opération dénommée éthénolyse. Cette méthode est mentionnée notamment dans l'article de Soon Hyeok Hong et al. « Prevention of Undesirable Isomerization during Olefin Metathesis » paru au Journal of American Society 2005,127,17160-17161 qui mentionne à cette occasion les risques de concurrence entre isomérisation et métathèse. Elle est également citée dans l'article de J. C. Mol « Catalytic metathesis of unsaturated fatty acid esters and oils » paru dans Topics in Catalysis Vol. 17, Nos 1-4, February 2004 pages 97 à 104*.*

On doit observer dans les méthodes ainsi décrites la formation de l'acide gras ω-insaturé à « chaîne courte » s'accompagne toujours de la formation concomitante d'une α-oléfine. Ceci présente l'inconvénient pour l'économie du procédé de devoir trouver sur place une utilisation industrielle pour cette α-oléfine sous produit de la réaction. En effet, le prix de marché des α-oléfines étant voisin de celui des acides gras mono-insaturés, la seule partie acide gras ω-insaturé doit sur le plan économique supporter la totalité du coût de synthèse.

Cette réaction est illustrée par le schéma ci-dessous illustrant l'éthénolyse de l'acide pétrosélinique.

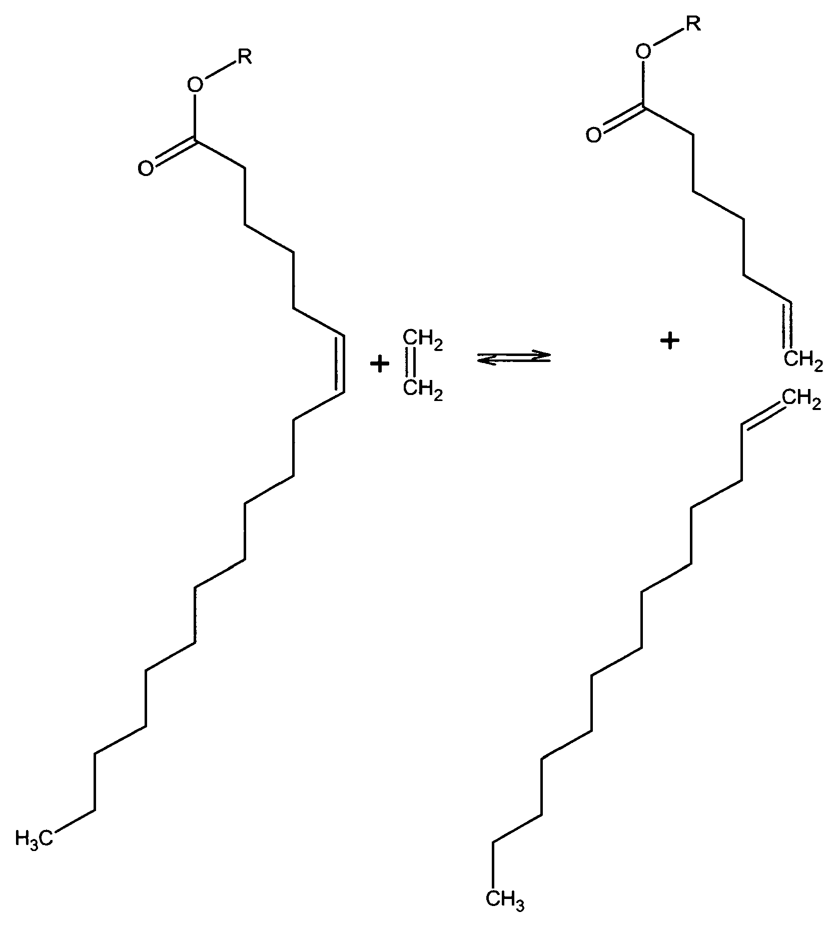

L'invention vise à s'affranchir de cet inconvénient en proposant un procédé fabriquant à partir d'acides ou d'esters gras d'origine naturelle uniquement des acides gras ω-insaturés à « chaîne courte ».

Dans le procédé de l'invention, on peut traiter l'acide gras soit sous sa forme acide soit sous sa forme ester. Le passage d'une forme à l'autre s'effectue par méthanolyse, estérification ou hydrolyse. Pour la facilité de l'exposé la suite de la description visera principalement l'acide sachant que la réaction est immédiatement transposable à l'ester.

La présente invention a pour objet un procédé de synthèse d'acides ou esters gras ω-insaturés à « chaîne courte » de formule générale CH₂=CH-(CH₂)n-COOR dans laquelle n est un nombre entier compris entre 2 et 11 et R soit H soit un radical alkyle comportant de 1 à 4 atomes de carbone, à partir d'acides ou esters gras mono-insaturés d'origine naturelle à longue chaîne de formule CH₃-(CH₂)ₘ-CH=CH-(CH₂)ₚ-COOR dans laquelle m et p sont des nombres entiers identiques ou différents compris entre 2 et 11, tels que la molécule comporte au moins 10 atomes de carbone adjacents dans la chaîne principale, consistant dans une première étape à soumettre la charge d'acides ou esters gras principalement mono-insaturés à longue chaîne à une oxydation par voie de fermentation conduisant à la formation de d'α-ω diacides à longue chaîne, puis dans une deuxième étape à soumettre le produit issu de la première étape à une métathèse croisée avec l'éthylène pour conduire à la formation de deux acides ou esters gras ω-insaturés à « chaîne courte ».

Dans le procédé de l'invention on entend par acides ou esters gras ω-insaturés à « chaîne courte » des composés comportant généralement un nombre d'atomes de carbone adjacents compris entre 5 et 14, ce nombre étant toujours inférieur au nombre d'atomes de carbone de la chaîne principale de l'acide ou ester gras mono-insaturé utilisé pour sa synthèse. La longueur de chaîne de l'acide oméga-insaturé rapportée à celle de l'acide gras insaturé de départ est comprise entre 0,2 et 0,8 et de préférence comprise entre 0,35 et 0,75, et encore plus de préférence entre 0,42 et 0,72, et encore plus de préférence entre 0,45 et 0,64. Dans une variante préférée du procédé de l'invention ces acides ou esters oméga-insaturés comporteront de 6 à 14 atomes de carbone par molécule.

Dans le procédé de d'invention on entend par charge d'acides ou esters gras principalement mono-insaturés, une charge ayant une teneur pondérale en acides ou esters gras monoinsaturés supérieure à 50 % et de préférence supérieure à 80 %.

Selon la charge utilisée et les conditions de l'oxydation par fermentation on obtient soit deux acides gras ω-insaturés à « chaîne courte » différents ou si le diacide formé est symétrique un seul acide gras ω-insaturé à « chaîne courte » comme produit final ce qui est particulièrement avantageux au niveau du rendement du procédé.

Dans le procédé de d'invention on entend par acides ou esters gras mono-insaturés de chaîne longue d'origine naturelle, un acide ou ester issu des milieux végétal ou animal, y compris les algues, plus généralement du règne végétal, et donc renouvelable comportant au moins 10 et de préférence au moins 14 atomes de carbone par molécule.

On peut citer à titre d'exemples de tels acides, les acides en C10, les acides obtusilique (cis-4-décénoique), les acides en C12, les acides lauroléique (cis-5-dodécénoique) et lindérique (cis-4-dodécénoique), les acides en C14, les acides myristoléique (cis-9-tétradécénoique), physétérique (cis-5-tetradécénoique) et tsuzuique (cis-4-tetradécénoique), l'acide en C16, l'acide palmitoléique (cis-9-hexadécénoique), les acides en C18, les acides oléique (cis-9-octadécénoique), élaïdique (trans-9-octadécénoique), pétrosélinique (cis-6-octadécénoique), vaccénique (cis-11-octadécénoique) et ricinoléique (12-hydroxy-cis-9-octadécénoique), les acides en C20, les acides gadoléique (cis-9-eicosénoique), gondoique (cis-11-eicosénoique), l'acide cis-5-eicosènoique et l'acide lesquérolique (14-hydroxy-cis-11-eicosénoique), les acides en C22, les acides cétoléique (cis-11-docosénoique) et érucique (cis-13-docosénoique).

La première étape est réalisée par fermentation au moyen d'un micro-organisme, tel qu'une bactérie, un champignon ou une levure permettant l'oxydation de l'acide ou ester gras de la charge. On utilise des micro-organismes contenant des enzymes de type *Oxygenase* susceptibles d'oxyder la charge en formant une fonction trivalente de type acide -COOH ou ester -COOR.

Cette fermentation pourra par exemple être réalisée en présence d'une souche de *Candida tropicalis* contenant les enzymes Cytochrome P450 Monooxygenase telles que celles décrites dans la publication de W. H. Eschenfeldt et al « Transformation of fatty Acids Catalyzed by Cytochrome P450 Monooxygenase Enzymes of Candida tropicalis » paru dans Applied and Environmental Microbiology, Oct. 2003 p. 5992-5999 et les brevets FR 2,445,374, US 4,474,882, US 3,823,070, US 3,912,586, US 6,660,505, US 6,569,670 et 5,254,466.

Les réactions de métathèse mises en oeuvre en deuxième étape sont connues, mais n'ont jamais été décrites, avec les diacides depuis longtemps même si leurs applications industrielles sont relativement limitées. On peut se référer à propos de leur utilisation dans la transformation des acides (esters) gras, à l'article de J.C. Mol « Catalytic metathesis of unsaturated fatty acid esters and oil » paru dans Topics in Catalysis Vol. 27, Nos. 1-4, February 2004 (Plenum Publishing Corporation).

La catalyse de la réaction de métathèse a fait l'objet de très nombreux travaux et le développement de systèmes catalytiques sophistiqués. On peut citer par exemple les complexes au tungstène développés par Schrock et al J. Am. Chem. Soc. 108 (1986) 2771, ou Basset et al. Angew. Chem., Ed. Engl. 31 (1992) 628. Plus récemment, sont apparus les catalyseurs dits de Grubbs (Grubbs et al. Angew. Chem., Ed. Engl. 34 (1995) 2039 et Organic Lett. 1 (1999) 953) qui sont des complexes ruthénium-benzylidène. Il s'agit de catalyse homogène. Il a été aussi développé des catalyseurs hétérogènes à base de métaux tels que rhénium, molybdène et tungstène déposés sur alumine ou silice. Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est d'augmenter la sélectivité de la réaction vis-à-vis des réactions parasites telles que les « homo-métathèses » entre les réactifs mis en présence. Ils portent non seulement sur la structure des catalyseurs mais également sur l'incidence du milieu réactionnel et les additifs pouvant être introduits.

Dans le procédé de l'invention, le catalyseur de métathèse actif et sélectif utilisé est à base de ruthénium et de rhénium.

La deuxième étape est illustrée par des exemples de synthèse de diacides gras à courtes chaînes ci après. Tous les mécanismes détaillés ci-dessous illustrent, pour faciliter l'exposé, la forme acide. Cependant, la métathèse est aussi efficace avec un ester et même souvent plus efficace. De la même manière, les schémas illustrent des réactions avec l'isomère cis des acides (ou esters); les mécanismes sont aussi bien applicables aux isomères trans.

Le processus réactionnel de cette deuxième étape mettant en oeuvre le diacide oléique, ou acide α-ω 9-octadécènedioique, et l'éthylène est le suivant : HOOC-(CH₂)₇-CH=CH-(CH₂)₇-COOH + CH₂=CH₂ ⇔ 2 CH₂=CH-(CH₂)₇-COOH

On peut observer que le procédé présente dans ce cas un avantage tout particulier dans la mesure où l'on évite la formation d'un co-produit pour fabriquer le seul acide 9-décènoique.

Le processus réactionnel est illustré par le schéma 1 ci-dessous.

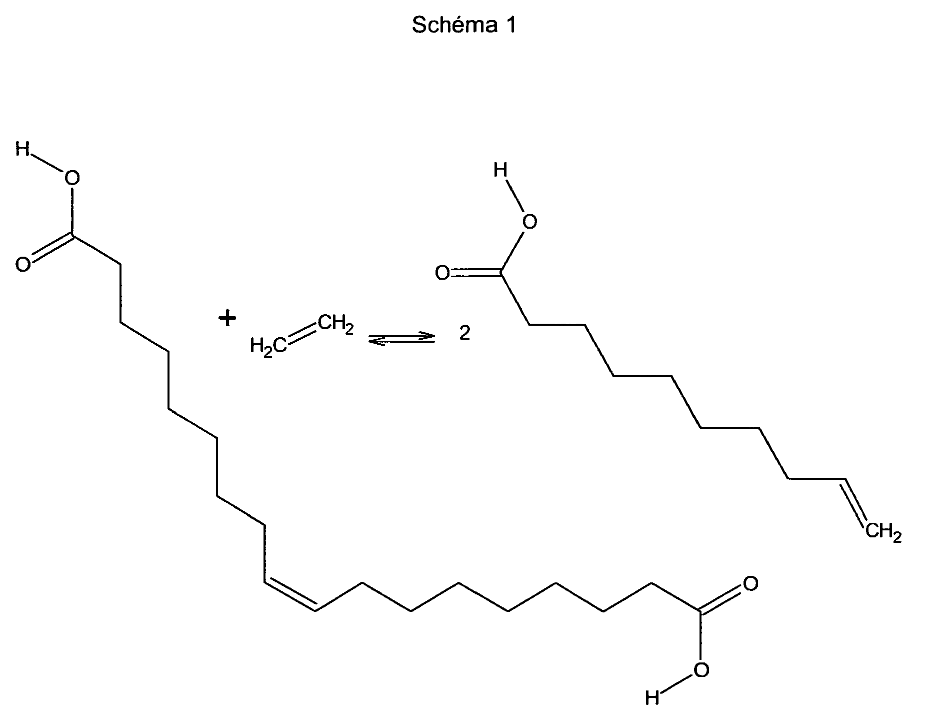

Dans certaines conditions de fermentation l'huile de macadamia ou de l'huile d'argousier est oxydée en partie en un diacide en C16, l'acide α-ω 7-hexadécènedioique à partir de l'acide palmitoléique contenu dans ces huiles.

Le processus réactionnel de cette deuxième étape mettant en oeuvre ce diacide et l'éthylène pour produire deux acides ω- insaturés différents, les acides 9-décènoique et 7-octènoique, peut être décrit par le mécanisme suivant :

HOOC-(CH₂)₅-CH=CH-(CH₂)₇-COOH + CH₂=CH₂ ⇔ CH₂=CH-(CH₂)₇-COOH + CH₂=CH-(CH₂)₅-COOH

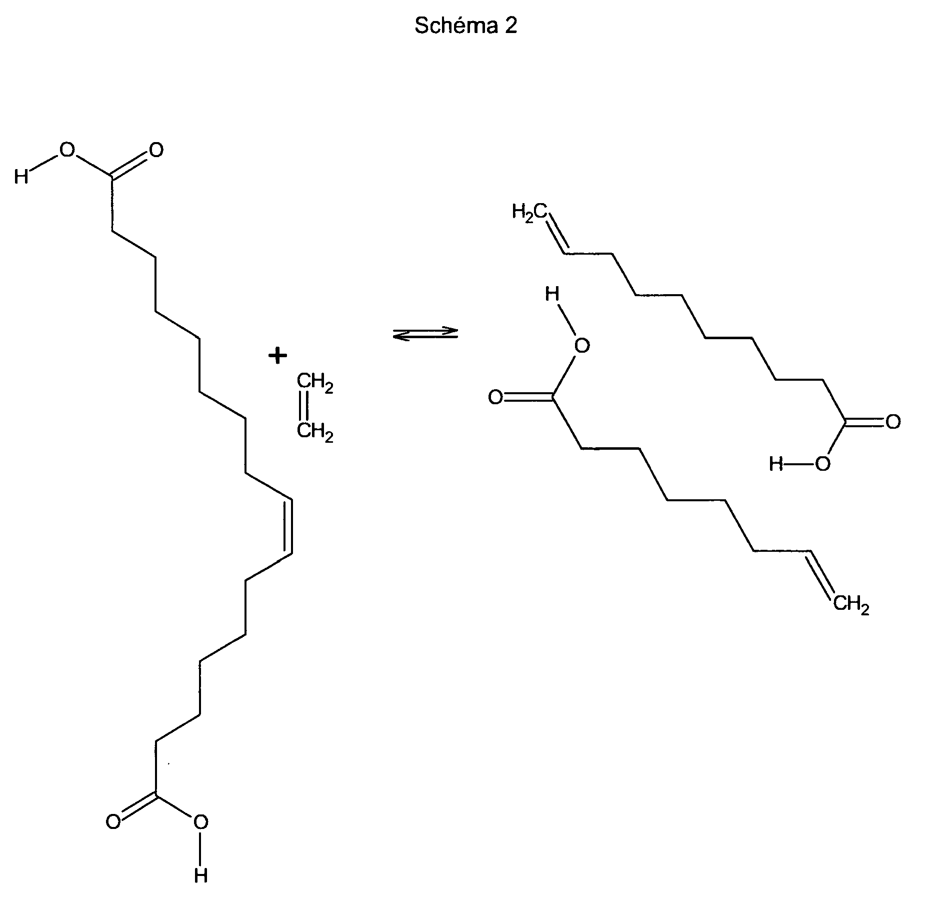

L'acide lauroléique est converti lors de la fermentation en l'acide α-ω 5-dodécènedioique. La deuxième étape du procédé va conduire à deux acides ω-oléfiniques de longueurs différentes, les acides 7-octènoique et 5-héxènoique, selon le processus suivant.

HOOC-(CH₂)₅-CH=CH-(CH₂)₃-COOH +CH₂=CH₂ ⇔ CH=CH₂-(CH₂)₅-COOH + CH₂=CH-(CH₂)₃-COOH

L'acide myristoléique est converti lors de la fermentation en l'acide α-ω 5-tetradécènedioique. La deuxième étape du procédé va conduire à deux acides w-oléfiniques de longueurs différentes, les acides 9-décènoique et 5-héxènoique selon le processus suivant.

HOOC-(CH₂)₇-CH=CH-(CH₂)₃-COOH + CH₂=CH₂ ⇔ CH₂=CH-(CH₂)₇-COOH + CH₂=CH-(CH₂)₃-COOH

L'invention est illustrée par les exemples ci-après.

### EXEMPLES

### Exemple 1 : Fermentation de l'acide oléique

Dans cet exemple on utilisera une levure contenant au moins une enzyme *Oxygénase.* La levure sera cultivée à pH=7, dans un milieu d'eau désionisée contenant du sorbitol, des oligoéléments, de l'urée et, et de l'acide oléique. Le mélange sera ensuite stérilisé à 120°C pendant 15 minutes. Une souche de levure sera ensuite inoculée au milieu de culture. La culture sera maintenue à 30°C. Une solution de soude sera ajoutée en continu pour maintenir le milieu à un pH de 7,0 à 7,5. Après 48 heures de culture, le diacide insaturé sera récupéré par extraction au diéthylether. Après élimination du solvant par évaporation, on récupèrera des cristaux qui après recristallisation auront un point de fusion de 69°C, c'est-à-dire équivalent à celui décrit pour le diacide 9-octadécènedioique.

### Exemple 2 : Fermentation de l'acide érucique

L'exemple 1 sera reproduit en mettant en oeuvre l'acide érucique. On obtiendra le diacide 9-docosènedioique.

### Exemple 3 : Métathèse croisée du diacide 9-octadécènedioique

Cet exemple illustre la synthèse de l'acide 9-décénoique à partir du diacide 9-octadécènedioique. Cette deuxième étape du procédé sera une métathèse croisée avec l'éthylène. On utilisera pour cette réaction un catalyseur complexe du type bispyridine ruthénium du type de celui décrit dans la publication de Chen-Xi Bai et al., Org. Biomol. Chem., (2005), 3, 4139-4142. La réaction sera effectuée dans CH₂Cl₂, à une concentration molaire d'éthylène deux fois supérieure à celle du diacide 9-octadécénedioique à une température de 80°C, et pendant 12 heures, en présence du catalyseur à une concentration de 1 % mole par rapport au diacide 9-octadécènedioique. Les rendements seront déterminés par analyse chromatographique et le rendement en acide 9-décénoique CH₂=CH-(CH₂)₇-COOH sera sensiblement équimolaire.

### Exemple 4 : Métathèse croisée du diacide 9-docosènedioique

On reproduira l'exemple 3 avec le diacide de l'exemple 2: diacide 9-docosènedioique. On obtiendra un rendement sensiblement équimolaire en acides 9-décèneoique et 13-tétradécéneoique.

### Exemple 5 : éthénolyse du diester C18 insaturé

Dans un tube de Schlenk de 50 ml purgé à l'azote, on charge 1 g de 9-octadécènedioate de méthyle (3 mmol), 47 mg (7,5.10-2 mmol) de catalyseur de Hoveyda-Grubbs de seconde génération (1,3-bis(2,4,6-triméthylphényl)-2-imidazolidinylidène)dichloro(o-isopropoxyphénylméthylène)ruthénium), disponible auprès de la société ALDRICH, et 15 ml de toluène distillé sur sodium-benzophénone. On ajoute 170 mg d'éthylène (6 mmol) et on laisse réagir 3 heures à 20°C sous agitation magnétique. Le mélange réactionnel est analysé par chromatographie en phase gaz. La conversion du diester unsaturé est de 83%. Le rendement en 9-décénoate de méthyle est de 64%

## Revendications

1. Procédé de synthèse d'acides ou esters gras ω-insaturés à chaîne courte de formule générale CH₂=CH-(CH₂)ₙ-COOR dans laquelle n est un nombre entier compris entre 2 et 11 et R soit H soit un radical alkyle comportant de 1 à 4 atomes de carbone, à partir d'acides ou esters gras mono-insaturés à longue chaîne de formule CH₃-(CH₂)ₘ-CH=CH-(CH₂)ₚ-COOR dans laquelle m et p sont des nombres entiers identiques ou différents compris entre 2 et 11, tels que la molécule comporte au moins 10 atomes de carbone adjacents dans la chaîne principale, consistant dans une première étape à soumettre la charge d'acides ou esters gras mono-insaturés à longue chaîne à une oxydation par voie de fermentation au moyen d'un micro-organisme contenant des enzymes de type *Oxygenase,* tel qu'une bactérie, un champignon ou une levure conduisant à la formation de d'α-ω diacides ou diesters à longue chaîne, puis dans une deuxième étape à soumettre le produit issu de la première étape à une métathèse catalytique croisée avec l'éthylène en présence de catalyseurs à base de ruthénium et de rhénium pour conduire à la formation de deux acides ou esters gras ω-insaturés à chaîne courte ayant une longueur de chaîne principale rapportée à celle de l'acide ou ester gras insaturé de départ compris entre 0,2 et 0,8 et de préférence 0,35 et 0,75.

2. Procédé selon la revendication 1 **caractérisé en ce que** la première étape est réalisée en présence d'une souche de *Candida tropicalis* contenant les enzymes Cytochrome P450 Monooxygenase.

3. Procédé selon l'une des revendications 1 ou 2 **caractérisé en ce que** l'on synthétise l'acide 9-décènoïque à partir de l'acide oléique.

4. Procédé selon l'une des revendications 1 à 3 **caractérisé en ce que** l'on synthétise les acides 9-décènoïque et 13-tetradécèneoique à partir de l'acide érucique.

## Claims

1. A method for the synthesis of short-chain ω-unsaturated fatty acids or esters of general formula CH₂=CH-(CH₂)ₙ-COOR in which n is an integer between 2 and 11 and R is H or an alkyl radical containing from 1 to 4 carbon atoms, from long-chain monounsaturated fatty acids or esters of formula CH₃-(CH₂)ₘ-CH=CH-(CH₂)ₚ-COOR in which m and p are integers, which may be identical or different, between 2 and 11, such that the molécule contains at least 10 adjacent carbon atoms in the main chain, said method comprising a first step of subjecting the long-chain monounsaturated fatty acid or ester charge to an oxidation by fermentation using a microorganism containing enzymes of *oxygenase* type, such as a bacterium, a fungus or a yeast, leading to the formation of long-chain α,ω-diacids or -diesters, and then a second step of subjecting the product resulting from the first step to catalytic cross-metathesis with ethylene in the presence of ruthenium- and rhenium-based catalysts so as to produce the formation of two short-chain ω-unsaturated fatty acids or esters having a main chain length, relative to that of the starting unsaturated fatty acid or ester, of between 0.2 and 0.8, and preferably 0.35 and 0.75.

2. The method as claimed in claim 1, **characterized in that** the first step is carried out in the presence of a strain of *Candida tropicalis* containing Cytochrome P450 Monooxygenase enzymes.

3. The method as claimed in either of claims 1 and 2, **characterized in that** 9-decenoic acid is synthesized from oleic acid.

4. The method as claimed in one of claims 1 to 3, **characterized in that** 9-decenoic acid and 13-tetradeceneoic acid are synthesized from erucic acid.

## Patentansprüche

1. Verfahren zur Synthese von kurzkettigen ω-ungesättigten Fettsäuren oder Fettsäureestern der allgemeinen Formen CH₂=CH-(CH₂)ₙ-COOR, worin n eine ganze Zahl im Bereich von 2 bis 11 ist und R entweder H oder eine Allylgruppe bedeutet, die 1 bis 4 Kohlenstoffatome umfasst, ausgehend von einfach ungesättigten, langkettigen Fettsäuren oder Fettsäureestern der Formel CH₃-(CH₂)ₘ-CH=CH-(CH₂)ₚ-COOR, worin in und p identische oder voneinander verschiedene ganze Zahlen, im Bereich von 2 bis 1 in einer Weise bedeuten, dass das Molekül in der Hautkette zumindest 10 benachbarte Kohlenstoffatome aufweist, das darin besteht, in einem ersten Schritt die Charge der einfach ungesättigten, langkettigen Fettsäuren oder Fettsäureester auf dem Wege der Fermentation mit Hilfe eines Mikroorganismus, der Enzyme von Typ *Oxygenase* umfasst, wie eines Bakteriums, eines Pilzes oder einer Hefe, einer Oxydation zu unterziehen, die zur Bildung von langkettigen α,ω-Disäuren oder α,ω-Disäureestern führt, und dann das Produkt aus dem ersten Schritt in einem zweiten Schritt in Gegenwart von Katalysatoren auf der Basis von Ruthenium und Rhenium einer katalytischen Kreuzmetathese mit Ethylen zu unterziehen, um zwei kurzkettige ω-ungesättigte Fettsäuren oder Fettsäureester zu bilden, deren Kettenlänge der Hauptkette im Vergleich mit der Kettenlänge der anfänglich eingesetzten ungesättigten Fettsäure oder des anfänglich eingesetzten ungesättigten Fettsäureestern im Bereich von 0,2 bis 0,8 und vorzugsweise 0,35 bis 0,75 liegt.

2. Verfahren nach Anspruch 1, **dadurch gekennzeichnet, dass** der erste Schritt in Gegenwart eines Stamms von *Candida tropicalis* durchgeführt wird, der die Enzyme Cytochrom P450-Monooxygenase umfasst

3. Verfahren nach einem der Anspruche 1 oder 2, **dadurch gekennzeichnet, dass** ausgehend von Ölsäure 9-Decensäure synthetisiert wird.

4. Vorfahren nach einem der Ansprüche 1 bis 3, **dadurch gekennzeichnet, dass** ausgehend von Erucasäure 9-Decensäure und 13-Tetradecensäure synthetisiert wird.
